# FASCICULE DE BREVET EUROPEEN

(11) **EP 0 560 691 B1**
(45) Date de publication et mention de la délivrance du brevet: **11.12.1996**
(21) Numéro de dépôt: 93420054.4
(22) Date de dépôt: 09.02.1993
(51) Int. Cl.: A61F 2/36

(54) **Tige fémorale pour prothèse de hanche à fixation primaire**
Schaftteil einer Hüftgelenkprothese zur primären Fixierung
Femoral stem for primary fixation hip prosthesis

(30) Priorité: 12.03.1992 FR 9203201
(43) Date de publication de la demande: 15.09.1993
(73) Titulaire: MERCK BIOMATERIAL FRANCE, 01800 Meximieux (FR)
(72) Inventeur: Collomb, Jean, F-26800 Porte les Valence (FR); Bejui, Jacques, F-69006 Lyon (FR); Majou, Claude Sous Montmert, F-01800 Meximieux (FR)
(74) Mandataire: Laurent, Michel

(56) Documents cités:
- EP-A- 0 190 981
- EP-A- 0 209 516
- FR-A- 2 600 526
- FR-A- 2 619 305
- FR-A- 2 638 086

## Description

L'invention concerne un nouveau type de tige fémorale perfectionnée pour prothèse de hanche à fixation primaire.

On connait depuis longtemps des tiges de prothèse à fixation primaire, c'est-à-dire ne faisant pas appel à du ciment pour leur fixation, et dont la caractéristique essentielle est d'être constituée par un fût à la périphérie externe duquel est taillé un filetage. Ainsi, on insère ces tiges par vissage dans l'os à prothéser.

Dans une première forme de réalisation, décrite dans le document US-A-4 693 724 des co-Demandeurs, la tige est filetée jusqu'à son extrémité. Cette solution, bien que largement répandue, présente l'inconvénient de créer en bout de tige une concentration de contraintes qui peut entraîner une corticalisation de toute ou partie du fût fémoral, pouvant être responsable de douleurs de la cuisse parfois importantes.

Dans le document FR-A-2 600 526, on a proposé de réaliser la tige en quatre portions distinctes, deux respectivement filetées, les deux extrêmes étant exemptes de filet.

Dans ces deux solutions, le pas de vis est moyen, c'est-à-dire est de l'ordre du centimètre, de sorte que une fois la tige en place, celle-ci a toujours une certaine tendance à se visser ou se dévisser, donc de bouger. Ces micro-scellements peuvent induire des déscellement mécaniques et/ou biologiques néfastes, et notamment des douleurs visibles lors des scintigraphies osseuses.

L'invention vise une tige fémorale perfectionnée filetée, facile à mettre en place et ne présentant pas cette facheuse tendance aux micro-vissages ou dévissages.

Cette tige fémorale pour prothèse de hanche à fixation primaire, qui comprend dans l'ordre:
. une tête, destinée à recevoir un col prothétique,
. une portion proximale en forme de tulipe évasée dont la périphérie présente un filetage définissant une rampe hélicoïdale,
. une portion distale cylindrique arrondie à son extrémité,
se caractérise en ce que le pas de la rampe hélicoïdale est au moins égal à la hauteur de la portion proximale.

En d'autres termes, la tige fémorale selon l'invention se distingue de l'état de la technique essentiellement par le fait que le pas de la rampe hélicoïdale du filetage externe, est au moins égal à la hauteur de cette portion proximale, c'est-à-dire de l'ordre de plusieurs, voire de la dizaine de centimètres, et non plus de l'ordre du centimètre, comme c'est le cas dans les prothèses actuelles. Cette caractéristique, combinée à la structure lisse cylindrique de la portion distale, évite avec succès les pics de contraintes, favorise la mise en place et évite les micro-mouvements.

Avantageusement, en pratique :
- la rampe hélicoïdale présente un pas qui est de l'ordre de grandeur du double de la hauteur de la portion proximale ;
- la portion proximale présente une section longitudinale en forme de tulipe évasée à rayon progressif variable et une section transversale circulaire ;
- le pas de vis présente une section trapézoïdale à pans coupés arrondis, dans lequel l'angle d'inclinaison de la face supérieure par rapport au plan de la section transversale, est supérieur à l'angle d'inclinaison de la face inférieure par rapport à ce même plan.

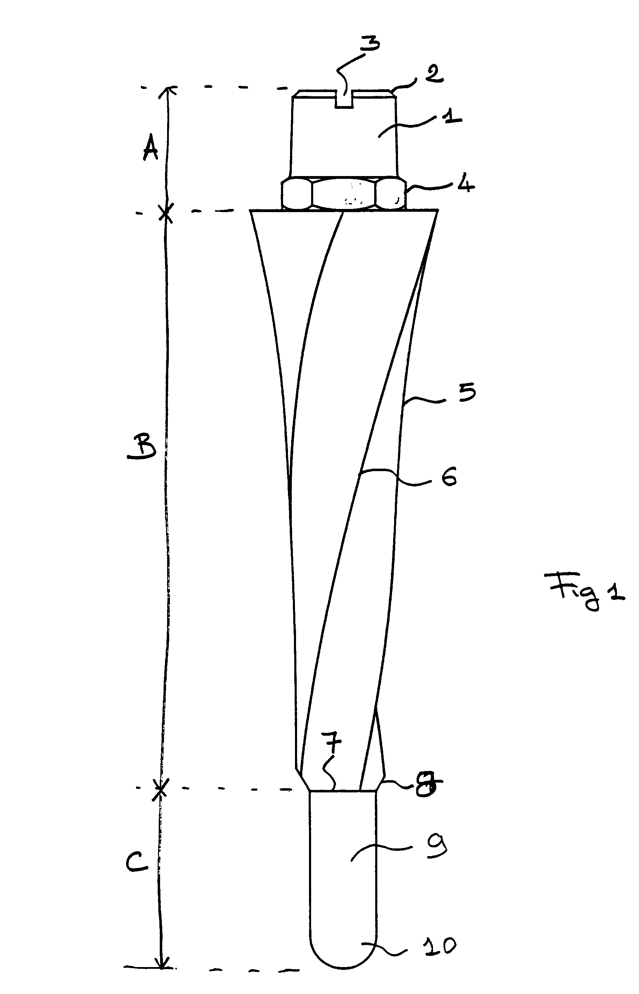

La manière dont l'invention peut être réalisée et les avantages qui en découlent, ressortiront mieux de l'exemple de réalisation qui suit à l'appui de la figure unique annexée qui montre, vue de côté, une tige fémorale caractéristique de l'invention.

Cette tige fémorale caractéristique comprend essentiellement trois parties distinctes successives, référencées (A), (B) et (C).

La tête (A) destinée à recevoir le col prothétique non représenté, comporte un cône de mors (1), chanfreiné (2), présentant en son milieu une entaille (3) et à sa base, une partie hexagonale régulière (4).

Cette tête (A) est solidaire d'une portion proximale (B) dont la génératrice (5) épouse la forme d'une tulipe évasée. Cette tulipe (5) présente un filetage (6) définissant une rampe hélicoïdale dont le pas est sensiblement égal au double de la hauteur de la portion proximale (B). Ce pas de vis présente, comme décrit dans le document EP-A-0 190 981 des co-Demandeurs correspondant au brevet américain US-A-4 693 724, une section trapézoïdale à pans coupés arrondis, dans laquelle l'angle d'inclinaison de la face inférieure par rapport au plan de la section transversale, est plus grand que l'angle d'inclinaison de la face supérieure par rapport à ce même plan, par exemple compris pour le premier entre 15 et 25°, de préférence au voisinage de 20°, alors que le second est compris entre 10 et 20°, de préférence voisin de 15°. La section longitudinale en forme de tulipe évasée de la portion proximale (B) est à rayon progressif variable, alors que la section transversale est avantageusement circulaire.

L'extrémité inférieure (7) de raccordement de la portion proximale caractéristique (B) avec la portion distale (C), présente un chanfrein (8) d'entrée dans la matière osseuse.

De manière connue, l'extrémité inférieure (7) de la portion proximale (B) est prolongée par la portion distale (C) constituée par une portion cylindrique (9) à extrémité (10) arrondie.

Dans une forme d'exécution pratique, la hauteur de la tête (A) est de l'ordre de dix-huit millimètres pour un diamètre voisin de quinze millimètres. La hauteur de la portion proximale (B) est de quatre vingt-huit millimètres pour un diamètre en haut de vingt-huit centimètres et en bas, juste à l'entrée du chanfrein (8) de quatorze millimètres. Le pas de la rampe hélicoïdale (6) est de cent cinquante (150) millimètres. La hauteur de la portion distale (C) est d'environ sept millimètres, le rayon de la portion arrondie de cinq millimètres et la hauteur de la portion chanfreinée (8) d'environ cinq millimètres.

En pratique, cette tige est métallique, notamment en titane, anodisée et microbrillée.

La mise en place d'une telle tige s'effectue non plus par vissage dans le canal prothétique fémoral préparé à cet effet, mais simplement par percusion, ce qui présente l'avantage d'éviter tout taraudage préalable de la corticale du fémur.

La tige fémorale conforme à l'invention présente de nombreux avantages par rapport aux tiges filetés à pas moyen commercialisées jusqu'alors. On peut citer:
- la rapidité de mise en place,
- la faible rotation, d'où autoblocage.

## Revendications

1. Tige fémorale pour prothèse de hanche à fixation primaire, qui comprend :
. une tête (A) destinée à recevoir un col prothétique,
. une portion proximale (B) en forme de tulipe évasée, dont la périphérie présente un filetage définissant une rampe hélicoïdale,
. une portion distale (C) cylindrique (9), arrondie à son extrémité (10),
***caractérisée*** en ce que le pas de la rampe hélicoïdale est au moins égal à la hauteur de la portion proximale (B).

2. Tige fémorale selon la revendication 1, ***caractérisée*** en ce que la portion proximale (B) présente une section longitudinale en forme de tulipe évasée à rayon progressif variable, et une section transversale circulaire.

3. Tige fémorale selon l'une des revendications 1 et 2, ***caractérisée*** en ce que le filetage (6) présente une section trapézoïdale à pans coupés arrondis, dans laquelle l'angle d'inclinaison de la face supérieure par rapport au plan de la section transversale, est supérieur à l'angle d'inclinaison de la face inférieure par rapport à ce même plan.

## Claims

1. Femoral shank for primary fixation hip prosthesis comprising:
- a head (A) intended to receive a prosthetic neck ;
- a proximal portion (B) which is tulip tapered in shape and which outer surface is helicoidally threaded ;
- a cylindrical distal portion (C) with a rounded hip (10),
characterized in that the pitch of the helicoidal threaded outer surface is at least equal to the height of the proximal portion (B).

2. Femoral shank according to claim 1, characterized in that the proximal portion (B) has a lengthwise section in the shape of a tapered tulip with a progressively variable radius of curvature, and a cross section circular in shape.

3. Femoral shank according to one of claims 1 and 2, characterized in that the thread has a trapezoïdal section with the corners cut off and rounded, in which the angle of inclination of the upper face with respect to the plane of the cross section is greater than the angle of inclination of its lower face in relation to the same plane.

## Patentansprüche

1. Femurschaft einer Hüftgelenkprothese zur primären Fixierung, bestehend aus:
. einem Kopf (A) für die Aufnahme eines Prothesenhalses,
. einem proximalen Abschnitt (B) in Form einer konisch erweiterten Tulpe, deren Rand ein Gewinde mit spiralförmiger Steigung beschreibt,
. einem zylindrischen distalen (C) Abschnitt (9), der am Ende (10) abgerundet ist,
dadurch gekennzeichnet, daß die Ganghöhe der spiralförmigen Steigung mindestens der Höhe des proximalen Abschnitts (B) entspricht.

2. Femurschaft nach Anspruch 1, dadurch gekennzeichnet, daß der proximale Abschnitt (B) einen Längsschnitt in Form einer konisch erweiterten Tulpe mit veränderlichem zunehmendem Radius und einen kreisförmigen Querschnitt aufweist.

3. Femurschaft nach einem der Ansprüche 1 und 2, dadurch gekennzeichnet, daß das Gewinde (6) einen trapezförmigen Abschnitt mit abgerundeten Schrägkanten aufweist, wobei der Neigungswinkel der oberen Fläche gegenüber der Querschnittebene größer ist als der Neigungswinkel der unteren Fläche gegenüber der gleichen Ebene.
